# EUROPEAN PATENT SPECIFICATION

(11) **EP 1 756 274 B1**
(45) Date of publication and mention of the grant of the patent: **20.08.2008**
(21) Application number: 05746319.2
(22) Date of filing: 07.06.2005
(51) Int. Cl.: C12N 9/20, C12N 15/55, C12Q 1/34, C12Q 1/44

(54) **LIPOLYTIC ENZYME ASSAY**
LIPOLYSEENZYMTEST
TEST D' ENZYME LIPOLYTIQUE

(30) Priority: 07.06.2004 DK 200400885
(43) Date of publication of application: 28.02.2007
(73) Proprietor: Novozymes A/S, 2880 Bagsvaerd (DK)
(72) Inventor: BJØRNVAD, Mads Eskelund, DK-1955 Frederiksberg C (DK); ABEL, Gernot, DK-2100 Copenhagen Ø (DK)
(86) International application number: PCT/DK2005/000375
(87) International publication number: WO 2005/121334

(56) References cited:
- WO-A-98/20337
- US-A1- 2003 166 298
- WILKES J G ET AL: "Sample preparation for the analysis of flavors and off-flavors in foods" JOURNAL OF CHROMATOGRAPHY, vol. 880, no. 1-2, June 2000 (2000-06), pages 3-33, XP004204838 ISSN: 0021-9673
- GUPTA RANI ET AL: "Lipase assays for conventional and molecular screening: An overview." BIOTECHNOLOGY AND APPLIED BIOCHEMISTRY, vol. 37, no. 1, February 2003 (2003-02), pages 63-71, XP002292137 ISSN: 0885-4513 cited in the application

## Description

### FIELD OF THE INVENTION

The present invention relates to a method for measuring the activity of a lipolytic enzyme and a method for screening a library of polypeptides for lipolytic enzyme of interest comprising using this method.

### BACKGROUND OF THE INVENTION

Lipolytic enzymes are enzymes capable of hydrolyzing ester bonds and they are widely used within different industries, such as the detergent industry and the baking industry.

A number of different assays are known for measuring the activity of lipolytic enzymes, which for example has been reviewed by Gupta R et al., 2003, Biotechnol. Appl. Biochem, 37, 63-71.

Since there is a continuous need for new and/or variants of old lipolytic enzymes in particular some which are adapted to an industrial applicability there is also a need for new assays capable of measuring the activity of said enzymes.

### SUMMARY OF THE INVENTION

The invention provides a method for measuring the activity of a lipolytic enzyme comprising measuring the pH of the gas phase of the enzymatic reaction between the lipolytic enzyme and a substrate, wherein the substrate comprises an ester bond between a C1-C10 carboxylic acid and an alcohol.

Furthermore, the present invention provides a method for screening a library of polypeptides for a lipolytic enzyme of interest comprising
a) measuring the pH of the gas phase of the enzymatic reaction between the polypeptides of the library and a substrate, wherein the substrate comprises an ester bond between a C1-C10 carboxylic acid and an alcohol
b) selecting a lipolytic enzyme of interest

### DETAILED DESCRIPTION OF THE INVENTION

### Definitions

The term "polypeptide" is in the context of the present invention intended to encompass oligopeptides, polypeptides and proteins as such.

The term "parent" is in the context of the present invention to be understood as a protein, which is modified to create a protein variant. The parent protein may be a naturally occurring (wild-type) polypeptide or it may be a variant thereof prepared by any suitable means. For instance, the parent protein may be a variant of a naturally occurring protein which has been modified by substitution, chemical modification, deletion or truncation of one or more amino acid residues, or by addition or insertion of one or more amino acid residues to the amino acid sequence, of a naturally-occurring polypeptide.

The term "variant" is in the context of the present invention to be understood as a protein which has been modified as compared to a parent protein at one or more amino acid residues.

The term "modification(s)" or "modified" is in the context of the present invention to be understood as to include chemical modification of a protein as well as genetic manipulation of the DNA encoding a protein. The modification(s) may be replacement(s) of the amino acid side chain(s), substitution(s), deletion(s) and/or insertions in or at the amino acid(s) of interest. Thus the term "modified protein", is to be understood as a protein which contains modification(s) compared to a parent protein.

### Lipolytic enzyme/ lipolytic enzyme of interest

The lipolytic enzyme/lipolytic enzyme of interest of the present invention is one that can hydrolyze ester bonds. Such enzymes include, for example, lipases, such as triacylglycerol lipase (EC 3.1.1.3), lipoprotein lipase (EC 3.1.1.34), monoglyceride lipase (EC 3.1.1.23), lysophospholipase and esterase (EC 3.1.1.1, EC 3.1.1.2). The numbers in parentheses are the systematic numbers assigned by the Enzyme Commission of the International Union of Biochemistry in accordance with the type of the enzymatic reactivity of the enzyme.

The lipolytic enzyme/lipolytic enzyme of interest may be prokaryotic, particularly a bacterial enzyme, e.g. from *Pseudomonas*. Examples are *Pseudomonas* lipases, e.g. from P. *cepacia* (US 5,290,694, pdb file 1OIL), *P. glumae* (N Frenken et al. (1992), Appl. En-vir. Microbiol. 58 3787-3791, pdb files 1TAH and 1QGE), *P. pseudoalcaligenes* (EP 334 462) and *Pseudomonas sp*. strain SD 705 (FERM BP-4772) (WO 95/06720, EP 721 981, WO 96/27002, EP 812 910). The *P. glumae* lipase sequence is identical to the amino acid sequence of *Chromobacterium viscosum* (DE 3908131 A1). Other examples are bacterial cutinases, e.g. from *Pseudomonas* such as *P. mendocina* (US 5,389,536) or *P. putida* (WO 88/09367).

Alternatively, the lipolytic enzyme/lipolytic enzyme of interest may be eukaryotic, e.g. a fungal lipolytic enzyme such as lipolytic enzymes of the *Humicola* family and the *Zygomycetes* family and fungal cutinases.

Examples of fungal cutinases are the cutinases of *Fusarium solani pisi* (S. Longhi et al., Journal of Molecular Biology, 268 (4), 779-799 (1997)) and *Humicola insolens* (US 5,827,719).

The *Humicola* family of lipolytic enzymes consists of the lipase from *H. lanuginosa* strain DSM 4109 and lipases having more than 50 % homology with said lipase. The lipase from *H. lanuginosa* (synonym *Thermomyces lanuginosus*) is described in EP 258 068 and EP 305 216, and has the amino acid sequence shown in positions 1-269 of SEQ ID NO: 2 of US 5,869,438.

The *Humicola* family also includes the following lipolytic enzymes: lipase from *Penicillium camembertii* (P25234), lipase/phospholipase from *Fusarium oxysporum* (EP 130064, WO 98/26057), lipase from *F. heterosporum* (R87979), lyso-phospholipase from *Aspergillus foetidus* (W33009), phospholipase A1 from *A. oryzae* (JP-A 10-155493), lipase from *A. oryzae* (D85895), lipase/ferulic acid esterase from *A. niger* (Y09330), lipase/ferulic acid esterase from *A. tubingensis* (Y09331), lipase from *A. tubingensis* (WO 98/45453), lysophospholipase from *A. niger* (WO 98/31790), lipase from *F. solanii* having an isoelectric point of 6.9 and an apparent molecular weight of 30 kDa (WO 96/18729).

The *Zygomycetes* family comprises lipases having at least 50 % homology with the lipase of *Rhizomucor miehei* (P19515). This family also includes the lipases from *Absidia reflexa, A. sporophora, A. corymbifera, A. blakesleeana, A. griseola* (all described in WO . 96/13578 and WO 97/27276) and *Rhizopus oryzae* (P21811). Numbers in parentheses indicate publication or accession to the EMBL, GenBank, GeneSeqp or Swiss-Prot databases.

### Library of polypeptides

The present invention also relates to a method for screening a library of polypeptides for a lipolytic enzyme of interest. In the context of the present invention the term "library of polypeptides" is to be understood as a collection of at least two different polypeptides; i.e. at least two polypeptides which differ at one or more amino acid positions, e.g. the number of amino acids in the polypeptides may be different or the amino acid(s) at a particular position may be different.

Typically, the library of polypeptides may be prepared by introducing a library of nucleic acid sequences encoding the library of polypeptides into a host cell capable of expressing the polypeptides. Due to the genetic degeneracy the number of different nucleic acid sequences in said library may be higher than the number of different polypeptides. In particular said library of nucleic acid sequences may encode variants of a parent lipolytic enzyme; i.e. polypeptides which differ at, at least one amino acid position compared to a parent lipolytic enzyme. Thus the screening method may be used to screen for variants of a parent lipolytic enzyme. Such variants may be produced by e.g. random mutagenesis or site-directed mutagenesis of a parent lipolytic enzyme or other well-known methods to a person skilled in the art. Thus in a particular embodiment the library of polypeptides may be a library of variants of parent lipolytic enzyme. Examples of suitable parent lipolytic enzymes include but are not limited to those mentioned above in the section of lipolytic enzymes. In particular the parent lipolytic enzyme may be Lipolase®, a lipase derived from *Humicola lanuginosa* described in EP 258 068 and EP 305 216, or a lipase from *Pseudomonas* or *Bacillus*.

In another embodiment said library of nucleic acid sequences may encode polypeptides derived from one or a number of different organisms. Thus the method may be used to screen one or a number of different organisms for expression of a lipolytic enzyme activity.
In another embodiment the library of polypeptides may be prepared by synthesizing the polypeptides.

Methods for preparing a library of nucleic acid sequences, introducing it into a host cell, expressing the polypeptides encoded by said library of polypeptides in the host cells and methods for synthesizing polypeptides are well known to a person skilled in the art and may e.g. be found in "Molecular cloning: A laboratory manual", Sambrook et al. (1989), Cold Spring Harbor lab., Cold Spring Harbor, NY; Ausubel, F. M. et al. (eds.); "Current protocols in Molecular Biology", John Wiley and Sons, (1995); Harwood, C. R., and Cutting, S. M. (eds.); "Molecular Biological Methods for Bacillus", John Wiley and Sons, (1990); "DNA Cloning: A Practical Approach, Volumes I and II", D.N. Glover ed. (1985); "Oligonucleotide Synthesis", M.J. Gait ed. (1984); "Nucleic Acid Hybridization", B.D. Hames & S.J. Higgins eds (1985); "Transcription And Translation", B.D. Hames & S.J. Higgins, eds. (1984); "Animal Cell Culture", R.I. Freshney, ed. (1986); "Immobilized Cells And Enzymes", IRL Press, (1986); "A Practical Guide To Molecular Cloning", B. Perbal, (1984).

### Substrate

The substrate of the present invention comprises an ester bond between a C1-C10 carboxylic acid and an alcohol.

In the context of the present invention a C1-C10 carboxylic acid is to be understood as a carboxylic acid of the general formula R-COOH, where R is a hydrocarbon with between 0-9 carbon atoms (C-atoms). A hydrocarbon is generally understood as a compound having only carbon and hydrogen, however R in the context of the present invention R may also be substituted, e.g. with a halogen.

R may be a saturated hydrocarbon which is also known as an alkane, i.e. a hydrocarbon comprising only single bonds, or it may be an unsaturated hydrocarbon, i.e. a hydrocarbon comprising one or more double or triple bonds. Hydrocarbons comprising a double bond are also known as alkenes, while hydrocarbons comprising a triple bond are known as alkynes. In most naturally occurring substrates the hydrocarbon is an alkane or an alkene.

R may be a linear or branched chain or it may be cyclic or aromatic.

The C1-C10 carboxylic acid of the present invention may be a C1, C2, C3, C4, C5, C6, C7, C8, C9 or a C10 carboxylic acid, in particular it may comprise between 2-8, e.g. 4-8 C-atoms.

In a particular embodiment of the present invention the R of the C1-C10 carboxylic acid is a linear, unsubstituted hydrocarbon. Examples of such suitable C1-C10 carboxylic acids include but are not limited to butyric acid (CH3(CH2)2COOH), valeric acid (CH3(CH2)3COOH), caproic acid (CH3(CH2)4COOH), and caprylic acid (CH3(CH2)6COOH) or capric acid (CH3(CH2)8COOH).

Carboxylic acids comprising a long hydrocarbon chain of the general formula CH3(CₓH_{y})COOH are generally known as fatty acids. Fatty acids are comprised in a number of different lipids wherein they are covalently bonded to an alcohol by an ester bond. Examples of such lipids include but are not limited to triacylglycerols (also known as triglycerides), waxes, digalactosyl diglycerides and phospholipids, where a triacylglycerol is a triester of three fatty acids and glycerol, a wax is a fatty acid esterified to a long chain alcohol and a phospholipid is a lipid comprising a phosphate group in the hydrophilic part of the lipid. All of these structures are well known to a person skilled in the art. Although, many naturally occurring lipids comprise carboxylic acids with a hydrocarbon chain with more than 10 C-atoms natural existing lipids comprising carboxylic acids with 10 or less C-atoms are known. The substrate of the present invention may be a lipid, e.g. one of those mentioned above; in particular it may be a triacylglycerol. Thus the alcohol of the substrate of the present invention may in particular be glycerol, glycerol-3-phosphate, a long chain alcohol or modified versions of these alcohols. Other examples of alcohols which may be present in the substrate are well known to a person skilled in the art.

The substrate of the present invention may be a natural occurring substrate, a synthetic substrate or a processed substrate, e.g. a naturally occurring substrate which has been processed by man. In the context of the present invention the term "natural occurring substrate" is to be understood as a compound which is present in nature.

Many lipids, both naturally occurring and those processed by man comprise a mixture of different triglycerides and fatty acids (sometimes described as free fatty acids as they are present as fatty acid and not as part of another compound e.g. a triglyceride). The composition of such lipids is often described by their content of the different fatty acids. In a particular embodiment of the present invention the substrate may be a mixture of one or more different triglycerides, a mixture of one or more different fatty acids, or a mixture of one or more different triglycerides and one or more different fatty acids. In particular the substrate of the present invention may be a fat or oil comprising a mixture of one or more different triglycerides and one or more different fatty acids, such as an animal fat, e.g. milk fat or butter, or a plant fat, e.g. olive oil. If the substrate comprises a mixture of different triglycerides and/or fatty acids it may in particular be a substrate comprising between 0.1-100 w/w%, such as between 0.5-100 w/w% or 1-95 w/w% or 1-85 w/w% or 1-75 w/w% or 1-50 w/w% or 1-25 w/w% or 5-95 w/w% or 5-75 w/w% or 5-50 w/w% or 5-25 w/w% or 10-95 w/w% or 10-75 w/w% or 10-50 w/w% or 10-25 w/w% or 15-95 w/w% or 15-75 w/w% or 15-25 w/w% or 20-95 w/w% or 20-75 w/w% or 20-50 w/w% or 25-95 w/w% or 25-75 w/w% or 25-50 w/w% or 40-100 w/w% or 40-80 w/w% or 60-80 w/w% or 50-100 w/w% or 50-75 w/w% or 60-100 w/w% or 60-80 w/w% or 70-100 w/w% or 70-95 w/w% or 70-80 w/w% of a C1-C10 carboxylic acid, such as one of those described above.

### Measuring pH

The pH of the gas phase may be measured by any known means for measuring pH. In a particular embodiment a pH indicator may be used to measure the pH, wherein the term "pH indicator" is to be understood as any means capable of indicating the pH. For example a pH indicator may be a compound which changes colour and/or flourescence at a certain pH. Typically the choice of pH indicator depends on the particular pH to be measured as the different pH indicators are typically only capable of indicating the pH in a particular pH interval. Examples of pH indicators which are compounds that change colours at a certain pH are well known to a person skilled in the art and include but are not limited to: Crystal Violet, Cresol Red, Thymol Blue, Erythrosin B, 2,4-dinitrophenol, Bromphenol Blue, Methyl Orange, Bromcresol Green, Methyl Red, Bromcresol Purple, Alizarin, Bromthymol Blue, Phenol Red, m-nitrophenol, o-Cresolphtalein, Phenolphthalein, Thymolphthalein, Alizarin Yellow R and Oregon Green® carboxylic acid (Molecular Probes). Furthermore, commercial pH indicators are available which are capable of indicating a broad spectrum of pH-values by colour and/or fluorescence.

The release of the C1-C10 carboxylic acid will typically due to their acidic nature lower the pH of the gas phase. Thus the depending on the pH of the gas phase before said acids enter it the pH indicator should in particular be capable of measuring or indicating a pH between 0-14, such as pH 2-12 or pH 3-12 or pH 3-10 or pH 3-9 or pH 4-12 or pH 4-10 or pH 5-12 or pH 5-10 or pH 5-8 or pH 6-12 or pH 6-10 or pH 6-8. In a particular embodiment of the present invention the pH indicator may be Bromcresol Green.

However, other pH indicators are envisioned to be used and may include but are not limited to fluorescent pH indicators.

Measuring the pH of the gas phase by the use of a pH indicator may be performed by e.g. visual or automatic inspection of the pH indicator. The pH indicator may be present in e.g. a liquid media or solid media or a gel, such as agarose. Means for measuring the absorbance or fluorescence are known to a person skilled in the art.

### Methods of the present invention

One embodiment of the present invention relates to a method for measuring the activity of a lipolytic enzyme comprising measuring the pH of the gas phase of the enzymatic reaction between the lipolytic enzyme and a substrate, wherein the substrate comprises an ester bond between a C1-C10 carboxylic acid and an alcohol.

The inventors of the present invention have found that the pH of the gas phase of the enzymatic reaction between a lipolytic enzyme and a substrate comprising an ester bond between a C1-C10 carboxylic acid and an alcohol can be used as a measurement for the activity of the lipolytic enzyme. As a result of the capability of the lipolytic enzyme to hydrolyse the ester bond of the substrate the C1-C10 carboxylic acid is released from the substrate. Due to the nature of lipolytic enzymes most assays for testing their activity are carried out at room temperature or slightly elevated temperatures, e.g. 10-80°C. At these temperatures the vapour pressure of the C1-C10 carboxylic acids is so that at least a fraction of the released C1-C10 carboxylic acid is capable of entering the gas phase. The inventors of the present invention have discovered that the released C1-C10 carboxylic acid or a fraction thereof which enters the gas phase may be detected by measuring the pH of the gas phase and that said pH correlates with the activity of the lipolytic enzyme. The effect the released C1-C10 carboxylic acid has on the pH of the gas phase depends of course of the pH of the gas phase before the reaction took place, however, as the C1-C10 carboxylic acid is an acid it may typically lower the pH of the gas phase as compared with the pH of the gas phase before the reaction took place.

The present invention also relates to a method for screening a library of polypeptides for a lipolytic enzyme of interest comprising
a) measuring the pH of the gas phase of the enzymatic reaction between the polypeptides of the library and a substrate, wherein the substrate comprises an ester bond between a C1-C10 carboxylic acid and an alcohol
b) selecting a lipolytic enzyme of interest

The criteria used for selecting a lipolytic enzyme of interest may generally be based on the measured pH of the gas phase of the enzymatic reaction between the lipolytic enzyme of interest and the substrate. For example the selection may be based on the actual pH, or on the difference between the measured pH and the pH of the gas phase of a control. Thus the selection of a lipolytic enzyme of interest may in particular be based on the change in pH of the gas phase generated by the enzyme when reacting with the substrate. The control may e.g. be the pH of the gas phase before the enzymatic reaction took place or it may be the pH of the gas phase of an enzymatic reaction of a known lipolytic enzyme where the conditions of the enzymatic reaction are the same as those used for screening the library of polypeptides. For example if the library represents variants of a parent lipolytic enzyme it may be relevant to use the pH of the gas phase of the enzymatic reaction between the parent lipolytic enzyme and the substrate as a control to select a variant (lipolytic enzyme of interest) for which the pH of the gas phase of the enzymatic reaction between the variant and the substrate is different from the pH created by the parent lipolytic enzyme. In particular it may be of interest to select a variant (lipolytic enzyme of interest) for which the pH of the gas phase is higher or lower than that for the parent lipolytic enzyme.

Other controls may be used depending on the criteria for selecting the lipolytic enzyme of interest.

A control may also be used when the method is used to measure the activity of a lipolytic enzyme. For example a so-called internal standard may be used, e.g. the pH generated by the lipolytic enzyme which is tested may be compared with the pH generated with a known lipolytic enzyme making it possible to correct for assay-assay variations, which is a well-known problem for a person skilled in the art. Such controls may also be used when screening a library of polypeptides for a lipolytic enzyme of interest.

The following is intended to encompass all methods of the present invention and reference to "enzymatic reaction" is intended to encompass both the enzymatic reaction between a lipolytic enzyme of the present invention and a substrate of the present invention, and the enzymatic reaction(s) between the polypeptides of the library of polypeptides of the present invention and a substrate of the present invention, however said library may comprise polypeptides which do not react with a substrate of the present invention.

The enzymatic reaction of the present invention may take place in a liquid solution. However, in a particular embodiment of the present invention the enzymatic reaction may take place on a textile. The gas phase is in the context of the present invention to be understood as the gas phase in contact with the phase in which the enzymatic reaction takes place. Thus for example if the enzymatic reaction takes place in a liquid solution the gas phase of the present invention is the gas phase which is in contact with said liquid solution and if the enzymatic reaction takes place on a textile it is the gas phase in contact with said textile. If the enzymatic reaction takes place in a closed container the gas phase will of course be defined by the container, however the enzymatic reaction of the present invention may also take place in an open container. If the enzymatic reaction takes place in an open container this may in particular be performed so that C1-C10 carboxylic acid released from the enzymatic reaction reacts with the pH indicator. As described above the pH indicator may in a particular embodiment be present in a liquid media or solid media or a gel, such as agarose. In this embodiment the C1-C10 carboxylic acids which are released by the enzymatic reaction between a lipolytic enzyme and a substrate of the present invention enter the gas phase above the phase in which said enzymatic reaction takes place and then subsequently enter the phase, e.g. the agarose, in which the pH indicator is present.

The pH may be measured at any time during the process of the enzymatic reaction, e.g. it may be measured before the enzymatic reaction is at equilibrium and/or before the released C1-C10 carboxylic acids are in equilibrium between the phase in which the enzymatic reaction took/takes place, e.g. a liquid phase, and the gas phase. When measuring the activity of an enzyme one is in principle measuring the reaction rate of the enzymatic reaction between the enzyme and its substrate. Thus when a control is used in the methods of the present invention and the pH is measured before the enzymatic reactions are at equilibrium it is of course important that the enzymatic reaction between the lipolytic enzyme/lipolytic enzyme of interest and the substrate and between the control and the substrate has had the same time to take place.

In practice a pH indicator may be present in the gas phase from the start of the enzymatic reaction and then after a period of time one may then read the pH indicator which may further be compared with the pH of a control.

If the enzymatic reaction takes place in solution said solution may comprise other components such as salts, pH stabilisers, detergents etc., which may ensure an optimal activity of the lipolytic enzyme/lipolytic enzyme of interest.

The enzymatic reaction between the lipolytic enzyme or the library of polypeptides and the substrate may take place in any suitable container, such as e.g. a shake flask, microtiter plates with e.g. 24 wells/plate, 96 wells/plate, 384 wells/plate, 1536 wells/plate or a higher number of wells per plate, or nanoliter well-less compartments. An advantage of using a microtiter plate is that it is generally easy to automate the detection procedure which is particularly useful when a library of polypeptides is screened.

In a particular embodiment of the present invention the enzymatic reaction between the lipolytic enzyme and the substrate takes place in one container which is in contact with a second container comprising a pH indicator. For example the enzymatic reaction may take place in one microtiter plate and the pH indicator may be present in another microtiter plate which is placed upside down on the microtiter plate comprising the enzymatic reaction. The pH of the gas phase may then be detected by visual or automatic inspection of the bottom (which is upward) of the microtiter plate comprising the pH indicator.

The methods of the present invention may be used in a number of different ways for example if the lipolytic enzyme or the library of polypeptides are expressed by a host cell it may be the culture broth of such cells which are used as a source of lipolytic enzyme/library of polypeptides. However, the lipolytic activity of a "pure" lipolytic enzyme may also be tested by the present invention, wherein the term "pure" is intended to encompass a sample of lipolytic enzyme comprising only a minor fraction of other components, such as a sample comprising at least 40w/w%, 50w/w%, 60w/w%, 70w/w%, 80w/w%, 90w/w%, 95w/w% or 95w/w% lipolytic enzyme.

In a particular embodiment the enzymatic reaction between a lipolytic enzyme or library of polypeptides and a substrate of the present invention may take place on a textile. Thus the methods of the present invention may prior to the enzymatic reaction, comprise the following steps:
i) contacting a lipolytic enzyme or library of polypeptides with a textile
ii) optionally rinsing the textile

The enzymatic reaction between the lipolytic enzyme or library of polypeptides and the substrate of the invention may in one embodiment take place by contacting the lipolytic enzyme or library of polypeptides with said enzyme after step i) or step ii), if the textile is rinsed. In another embodiment the enzymatic reaction may take place by contacting the substrate of the present invention with a textile and optionally rinsing the textile, prior to contacting it with a lipolytic enzyme or a library of polypeptides. Thus in a particular embodiment the methods of the present invention may comprise prior to the enzymatic reaction the following steps:
i) contacting a lipolytic enzyme or library of polypeptides with a textile, wherein said textile has been contacted with a substrate comprising an ester bond between a C1-C10 carboxylic acid and an alcohol and optionally rinsed prior to contacting it with the lipolytic enzyme or library of polypeptides
ii) optionally rinsing the textile

In a particular embodiment of the present invention this method may be carried out by having a substrate which does not comprise an ester bond between a C1-C10 carboxylic acid and an alcohol, such as lard, present on a textile. The textile may then be contacted with a lipolytic enzyme and optionally subsequently rinsed. The amount of residual lipolytic enzyme on the textile may then be measured by contacting said lipolytic enzyme present on the textile with a substrate comprising an ester bond between a C1-C10 carboxylic acid and an alcohol and measuring the pH of the gas phase of said enzymatic reaction as described above. The pH may then be used as a measurement of the activity of the lipolytic enzyme present on the textile.

In a particular embodiment the lipolytic enzyme or library of polypeptides is contacted with the textile by contacting the textile with a solution comprising a detergent and the lipolytic enzyme or library of polypeptides. In particular this may be performed under conditions similar to those of washing clothes, e.g. in the presence of mechanical stress such as described in WO 02/42740.

Many C1-C10 carboxylic acids have what most people would recognize as a bad smell. Thus, the method of the present invention may also be used to measure the bad smell which is released into the gas phase by the reaction between a lipolytic enzyme and a substrate comprising an ester bond between a C1-C10 carboxylic acid and an alcohol. When lipolytic enzymes are used in detergents it is particularly useful that bad smell is not released from the clothes after washing. Thus the method of the present invention may in particular be used to screen for lipolytic enzymes which e.g. release more or less C1-C10 carboxylic acids upon reaction with a substrate than a particular control enzyme.

### Textile

In the context of the present invention the term "textile" includes fabrics, garments, and yarns.

Fabric can be constructed from fibers by weaving, knitting or non-woven operations. Weaving and knitting require yarn as the input whereas the non-woven fabric is the result of random bonding of fibers (paper can be thought of as non-woven). In the present context, the term "fabric" is also intended to include fibers and other types of processed fabrics.

Woven fabric is constructed by weaving "filling" or weft yarns between wrap yarns stretched in the longitudinal direction on the loom. The wrap yarns must be sized before weaving in order to lubricate and protect them from abrasion at the high speed insertion of the filling yarns during weaving. The filling yarn can be woven through the warp yarns in a "over one - under the next" fashion (plain weave) or by "over one - under two" (twill) or any other myriad of permutations. Strength, texture and pattern are related not only to the type/quality of the yarn but also the type of weave. Generally, dresses, shirts, pants, sheeting's, towels, draperies, etc. are produced from woven fabric.

Knitting is forming a fabric by joining together interlocking loops of yarn. As opposed to weaving which is constructed from two types of yarn and has many "ends", knitted fabric is produced from a single continuous strand of yarn. As with weaving, there are many different ways to loop yarn together and the final fabric properties are dependent both upon the yarn and the type of knit. Underwear, sweaters, socks, sport shirts, sweat shirts, etc. are generally derived from knit fabrics.

Non-woven fabrics are sheets of fabric made by bonding and/or interlocking fibers and filaments by mechanical, thermal, chemical or solvent mediated processes. The resultant fabric can be in the form of web-like structures, laminates or films. Typical examples are disposable baby diapers, towels, wipes, surgical gowns, fibers for the "environmental friendly" fashion, filter media, bedding, roofing materials, backing for two-dimensional fabrics and many others.

The textile used in the present invention may be any known textile (woven, knitted, or non-woven). In particular the textile may be a cellulose-containing or cellulosic textile, such as cotton, viscose, rayon, ramie, linen, lyocell (e.g., Tencel, produced by Courtaulds Fibers), or mixtures thereof, or it may be a synthetic textile such as one of polyester, polyamic or nylon or mixtures of these or a mixture of cellulose-containing or cellulosic fibres and synthetic fibres. Another example of a suitable textile is one comprising other natural fibers such as wool and silk or mixtures of these or mixtures of these and one or more of the above mentioned fibres. Examples of mixtures of fibres include but are not limited to viscose/cotton blends, lyocell/cotton blends, viscose/wool blends, lyocell/wool blends, cotton/wool blends; flax (linen), ramie and other fabrics based on cellulose fibers, including all blends of cellulosic fibers with other fibers such as wool, polyamide, acrylic and polyester fibers, e.g. cotton/polyester blends, viscose/cotton/polyester blends, wool/cotton/polyester blends, flax/cotton blends etc. The term "wool," means any commercially useful animal hair product, for example, wool from sheep, camel, rabbit, goat, llama, and known as merino wool, Shetland wool, cashmere wool, alpaca wool, mohair, etc. and includes wool fiber and animal hair. The textile may be bleached, dyed or undyed. The term "polyester" refers to poly(ethylene terephthalate) which is synthesized by condensation, drawn into fibers from a melt, possibly cut to stables, possibly mixed with other fiber types, and spun to yarn. The yarn is dyed and knitted into cloth or made into carpets, or the yarn is woven into fabric and dyed.

The ability of an enzyme to bind or adhere to a textile depends both on the particular enzyme but also on the type of textile. For example it is generally more difficult to remove a lipolytic enzyme from a textile made of polyester than one made of cotton during rinsing as lipolytic enzymes may tend to bind and adhere stronger to hydrophobic materials than to more hydrophilic materials.

### Rinsing

In the context of the present invention the term "rinsing" is to be understood as contacting the fabric with a water-based solution and subsequently removing said solution, wherein the term "water-based solution" is to be understood as a solution comprising of a maximum of 20 w/v% other components than water, such as a maximum of 10 w/v% or 5 w/v% or 3 w/v% or 2 w/v% or 1 w/v% or 0.5 w/v% other components than water. Examples of such other components are given below.

As the methods of the present invention may be used to mimic the process of washing clothes the rinsing may in particular mimic the conditions of rinsing during washing of clothes. Generally, clothes are rinsed with water; however the composition of the water may vary depending on the source of the water, e.g. it may be river water, spring water or ground water. Furthermore, the geographical location of the source of water may affect its composition.

The hardness (total hardness) of a given source of water is due to its content of salts of the alkaline earth metals: calcium, magnesium, strontium and barium. Since strontium and barium are generally present in water only in traces, the hardness of water is defined as the content of calcium ions (Ca²+) and magnesium ions (Mg²+), The conventional procedure is to relate the statement of the water hardness only to calcium, in other words to express also the content of magnesium ions as calcium content. A practical measurement unit for the hardness that is frequently employed is the so-called German degree, which is defined as follows
1°dH =10 mg/I CaO.

"Hard" water is water that contains high concentrations of calcium carbonate and other minerals.

"Soft" water is water that contains low concentrations of calcium carbonate and other minerals.

Examples of other components which may be present in the water-based solution used for rinsing include but are not limited to buffers, especially buffers with a pH between 4-10, salts, softeners and small amounts of detergent. There may in particular be small amounts of detergent present if contacting the textile with the enzyme/library of polypeptides has been performed in the presence of a detergent, e.g. by "washing" of the textile. Examples of suitable buffers include but are not limited to those described below in table 1.

**Table 1:**

| pKa (20°C) | Buffer | pH range |
|---|---|---|
| 6.15 | MES | 5.5-7.0 |
| 6.46 | Bis-Tris | 5.7 - 7.3 |
| 6.6 | ADA | 5.8 - 7.4 |
| 6.8 | PIPES | 6.1 - 7.5 |
| 6.9 | ACES | 6.0 - 7.5 |
| 6.95 | MOPSO | 6.2 - 7.4 |
| 6.15 | BES | 6.6 - 8.0 |
| 7.2 | MOPS | 6.5 - 7.9 |
| 6.5 | TES | 6.8 - 8.2 |
| 7.55 | HEPES | 6.8 - 8.2 |
| 7.6 | DIPSO | 6.9 - 8.1 |
| 7.7 | TAPSO | 7.0 - 8.2 |
| 7.85 | POPSO | 7.2 - 8.5 |
| 9.9 | HEPPSO | 7.4 - 8.6 |
| 8 | EPPS | 7.5 - 8.5 |
| 8.15 | Tricine | 7.8 - 8.8 |
| 8.35 | Bicine | 7.7 - 9.1 |
| 8.4 | TAPS | 7.7 - 9.1 |
| 9.5 | CHES | 8.6 - 10.0 |
| 10 | CAPSO | 9.3 - 10.7 |
| 10.4 | CAPS | 9.7 - 11.0 |

Examples of salts or ions which may be present in water-based solution besides the Ca²⁺ and Mg²⁺ ions described above include but are not limited to NaCl, KCI, Strontium and barium.

Softeners are generally used during washing to improve the feel and freshness of the clothes and to reduce static electricity buildup (see e.g. Levinson MI, 1999, Journal of Surfactants and Detergents, 2, 223-235). One of the main ingredients in softeners is cationic tensides or surfactants, e.g. diamidoamine or diester quaternary or a triethanolamine-based esterquat, however, it may also comprise other ingredients such as perfumes, preservatives, buffers, dyes, optical brighteners, enzymes, dye stabilizers, ultraviolet light absorbers, chlorine scavengers and/or electrolytes (see e.g. Levinson MI, 1999, Journal of Surfactants and Detergents, 2, 223-235).

### Container

Also disclosed is a container comprising at least two parts, wherein one part comprises a pH indicator and the other part is suitable for containing a liquid. Said container may have any physical form and may be made of any material. In particular the container may have a physical form which makes it possible to read the pH indicated by the pH indicator from the outside of the container, e.g. the part of the container comprising a pH indicator may in particular be made of a transparent material. Said container may also in a particular embodiment have a physical form which makes it possible to automatically read the pH indicated by the pH indicator, e.g. by the use of apparatus capable of measuring absorbance or fluorescence depending on the choice of pH indicator. In a particular embodiment said container may comprise a multitude of containers each comprising two parts, wherein one part comprises a pH indicator and the other part is suitable for containing a liquid. More particularly, said multitude of containers may have a physical shape as two microtiter plates placed on top of each other with one plate being faced upside down so that the wells of each plate faces each other. If the container comprises a multitude of container means for avoiding that the gas from one container enters another may in particular be used. For example a piece of material may be placed between the two parts of the container, wherein said material has holes corresponding to each individual container. This may be as described in the examples where a piece of parafilm is placed over a first microtiter plate, wherein said parafilm comprises holes which are placed in connection with each of the well-openings of the microtiter plate, and then a second microtiter plate is placed upside-down on the first microtiter plate so that the well-openings of the first and second microtiter plate are situated in connection with each other. Thus each well-opening of the first microtiter plate will be in contact with a well-opening of the second microtiter plate, while the areas between the well-openings of the first microtiter plate will be sealed off from the similar areas of the second microtiter plate by the parafilm.

However, containers with other shapes and/or containers made of other materials may be used.

### MATERIALS AND METHODS

### Lipolytic enzymes

Lipolase® is a lipase derived from *Humicola lanuginosa* described in EP 258 068 and EP 305 216.
Lipex® is a variant of Lipolase® and described in WO 0060063.

### Media

SC = Synthetic complete medium.

### Methods

### Micro-laundry

Textile swatches stained with lard or butter were punched into wells of a 96 well microtiterplate. 150 µl of detergent (100 mM L-arginine) was dispensed into each well. 10 µl supernatant of yeast cells expressing the enzyme to be tested (grown in microtiterplates for 3-4 days in SC medium) was added to each well and the microtiter plates were incubated for 20 min at 30°Celcius at 500 rpm. The wash water was removed by a plate washer and the swatches were subsequently rinsed as described below.

### Rinsing

After performing the micro-laundry assay (described above) with different enzyme concentrations, the textile swatches present in the microwells were rinsed using artificially made water with a hardness of 15°dH (see materials and methods).

The rinsing process was performed by adding 180 microl water with a hardness of 15°dH, placing the plate on a orbital shaker set at 300 rpm for 5 min before removing the rinse water. This rinse process was repeated 3 times in total. After the final removal of rinse water a lipase substrate was added to the wells to measure the activity of lipase present on each textile-swatch.

### pH indicator plates (Bromocresolgreen-agarose)

1 gram of Agarose (Gibco BRL, Life technologies) was dissolved in 100 ml of 0.1mM Disodium Tetraborate Decahydrate (#0268 JT Baker, Mallinckrodt Baker, Deventer, Holland). The solution was briefly heated in a microwave oven until all the agarose was melted. After cooling to 55°C, bromocresolgreen was added to a final concentration of 0,006 % in 0.1 mM Na-borate.
Two different plates with Bromocresolgreen-agarose were prepared:
a) 80 Microl to each well of Polystyren microwell plates (normal size 96-well microtiter plate)
b) 15 Microl to each well of a small volume microbatch plate

The pH was read by transferring the plates to a spectrophotometer and measuring the absorbance of each well of the plate at 614 nm.

High absorbance at 614 nm indicates that the pH is unaltered, while low absorbance at 614 nm indicates that the pH has been lowered, i.e. is more acidic.

As the washed textile may contain minute amounts of lipase after the washing and rinsing it may be able to degrade butter, which is still also present at the textile, into fatty acids. Some of these fatty acids are volatile and will evaporate from the textile through the well of the wash plate into the opposing plate (the pH indicator plate) containing the bromocresolgreen-agarose. The free fatty acid will enter the buffer and due to its acidic properties will lower the pH and the indicator will change accordingly.

### EXAMPLES

### Example 1

### Measurement of the release of short fatty acids from textile swatches washed with different lipases

Textile swatches stained with butter and Sudanred were placed in the wells of a 96- well microtiter plate. The swatches were washed according to the micro-laundry assay (described above) with Lipase®, Lipex®, Var3 or Var5 at a concentration of 0, 0.12, 0.25, 0.5, 1 or 2 ppm enzyme and subsequently rinsed as described above. This rinse process was repeated 3 times in total. After the final removal of rinse water a piece of Parafilm with holes corresponding to the opening of each well were placed on top of the 96-well plate comprising the washed and rinsed textile swatches.

One pH indicator plate containing 80 Microl Bromocresolgreen-agarose/well (a) and one containing 15 Microl Bromocresolgreen-agarose/well (b) where each placed upside down on top of a 96-well plate comprising the washed and rinsed textile swatches. The plates were placed so that the Parafilm with the 96 holes were placed as a leakage seal between the textile plate and the indicator plate. This plate sandwich was mounted with approximately 2 kg of weight to keep a good sealing so any evaporating compounds were transferred vertically to the perpendicular well on the indicator plate and not horizontally to other indicator wells. After 18 hours incubation at room temperature the absorbance at 614 nm of the wells with bromocresolgreen-agarose were measured in a spectrophotometer. The results are shown below in table 2.

**Table 2:**

| Lipase ppm | Lipolase® | Lipex® | Var3 | Var5 |
|---|---|---|---|---|
| 0 | 1,0757 | 1,078 | 1,0155 | 1,0709 |
| 0.12 | 1,1182 | 0,7963 | 0,5228 | 0,8043 |
| 0.25 | 1,0171 | 0,6243 | 0,4345 | 0,609 |
| 0.5 | 0,9394 | 0,5373 | 0,3386 | 0,4625 |
| 1 | 0,806 | 0,4453 | 0,2796 | 0,3833 |
| 2 | 0,5774 | 0,3579 . | 0,2398 | 0,3103 |

The results show that both Var3 and Var5 lower the pH of the gas phase more than both Lipolase® and Lipex®.

In a similar set-up the amount of released C1-C10 carboxylic acids were measured by Gas chromatography which showed the similar results as above.

## Claims

1. A method for measuring the activity of a lipolytic enzyme comprising measuring the pH of the gas phase of the enzymatic reaction between the lipolytic enzyme and a substrate, wherein the substrate comprises an ester bond between a C1-C10 carboxylic acid and an alcohol.

2. A method for screening a library of polypeptides for a lipolytic enzyme of interest comprising
a) measuring the pH of the gas phase of the enzymatic reaction between the polypeptides of the library and a substrate, wherein the substrate comprises an ester bond between a C1-C10 carboxylic acid and an alcohol
b) selecting a lipolytic enzyme of interest.

3. A method according to any of the preceding claims, wherein the C1-C10 carboxylic acid is selected from the group consisting of: butyric acid, valeric acid, caproic acid and caprylic acid.

4. A method according to any of the preceding claims, wherein the alcohol is glycerol.

5. A method according to claim 4, wherein the substrate is a triglyceride.

6. A method according to any of the preceding claims, wherein the substrate is a mixture of triglycerides and fatty acid, e.g. milk fat, butter, olive oil.

7. A method according to any of the preceding claims, wherein the lipolytic enzyme or lipolytic enzyme of interest is a lipase (E.C. 3.1.1.3).

8. A method according to any of the preceding claims, wherein the substrate is present on a textile.

9. A method according to any of the preceding claims, wherein, prior to the enzymatic reaction, said methods comprise the following steps:
i) contacting the lipolytic enzyme or library of polypeptides with a textile, and
ii) optionally rinsing the textile.

## Patentansprüche

1. Verfahren zum Messen der Aktivität eines lipolytischen Enzyms, umfassend das Messen des pH der Gasphase der enzymatischen Reaktion zwischen dem lipolytischen Enzym und einem Substrat, wobei das Substrat eine Esterbindung zwischen einer C1-C10-Carbonsäure und einem Alkohol umfasst.

2. Verfahren zum Screenen einer Bibliothek von Polypeptiden auf ein lipolytisches Enyzm von Interesse, umfassend
a) Messen des pH der Gasphase der enzymatischen Reaktion zwischen den Polypeptiden der Bibliothek und einem Substrat, wobei das Substrat eine Esterbindung zwischen einer C1-C10-Carbonsäure und einem Alkohol umfasst;
b) Auswählen eines lipolytischen Enzyms von Interesse.

3. Verfahren nach einem beliebigen der vorhergehenden Ansprüche, wobei die C1-C10-Carbonsäure ausgewählt wird aus der Gruppe bestehend aus: Buttersäure, Valeriansäure, Capronsäure und Caprylsäure.

4. Verfahren nach einem beliebigen der vorhergehenden Ansprüche, wobei der Alkohol Glycerin ist.

5. Verfahren nach Anspruch 4, wobei das Substrat ein Triglycerid ist.

6. Verfahren nach einem beliebigen der vorhergehenden Ansprüche, wobei das Substrat ein Gemisch von Triglyceriden und Fettsäure, wie Milchfett, Butter, Olivenöl, ist.

7. Verfahren nach einem beliebigen der vorhergehenden Ansprüche, wobei das lipolytische Enzym oder das lipolytische Enzym von Interesse eine Lipase (E.C. 3.1.1.3) ist.

8. Verfahren nach einem beliebigen der vorhergehenden Ansprüche, wobei das Substrat auf einem Gewebe vorhanden ist.

9. Verfahren nach einem beliebigen der vorhergehenden Ansprüche, wobei, vor der enzymatischen Reaktion, die Verfahren die folgenden Schritte umfassen:
(i) das Inkontaktbringen des lipolytischen Enzyms oder der Bibliothek von Polypeptiden mit einem Gewebe, und
(ii) wahlweise das Spülen des Gewebes.

## Revendications

1. Méthode pour mesurer l'activité d'une enzyme lipolytique comprenant la mesure du pH de la phase gazeuse de la réaction enzymatique entre l'enzyme lipolytique et un substrat, dans laquelle le substrat comprend une liaison ester entre un acide carboxylique en C1-C10 et un alcool.

2. Méthode de criblage d'une banque de polypeptides pour une enzyme lipolytique d'intérêt comprenant :
a) la mesure du pH de la phase gazeuse de la réaction enzymatique entre les polypeptides de la banque et un substrat, dans laquelle le substrat comprend une liaison ester entre un acide carboxylique en C1-C10 et un alcool ;
b) la sélection d'une enzyme lipolytique d'intérêt.

3. Méthode selon l'une quelconque des revendications précédentes, dans laquelle l'acide carboxylique en C1-C10 est sélectionné parmi le groupe consistant en : l'acide butyrique, l'acide valérique, l'acide caproïque, et l'acide caprylique.

4. Méthode selon l'une quelconque des revendications précédentes, dans laquelle l'alcool est le glycérol.

5. Méthode selon la revendication 4, dans laquelle le substrat est un triglycéride.

6. Méthode selon l'une quelconque des revendications précédentes, dans laquelle le substrat est un mélange de triglycérides et d'acide gras, par ex. matière grasse du lait, beurre, huile d'olive.

7. Méthode selon l'une quelconque des revendications précédentes, dans laquelle l'enzyme lipolytique ou l'enzyme lipolytique d'intérêt est une lipase (E.C. 3.1.1.3).

8. Méthode selon l'une quelconque des revendications précédentes, dans laquelle le substrat est présent sur un textile.

9. Méthode selon l'une quelconque des revendications précédentes, dans laquelle, avant la réaction enzymatique, lesdites méthodes comprennent les étapes suivantes :
i) la mise en contact de l'enzyme lipolytique ou de la banque de polypeptides avec un textile, et
ii) facultativement, le rinçage du textile.
